(19) Europäisches Patentamt
European Patent Office
Office européen des brevets

(11) Veröffentlichungsnummer: **0 002 042**
**B1**

(12) # EUROPÄISCHE PATENTSCHRIFT

(21) Anmeldenummer: **78101351.1**

(22) Anmeldetag: **11.11.78**

(51) Int. Cl.³: **C 07 D 413/10,** C 07 D 417/10, C 07 D 263/56, C 07 D 251/18, C 07 D 277/66, D 06 L 3/12

(54) **Diamino-1,3,5-triazinylstilbenverbindungen, Verfahren zu deren Herstellung und ihre Verwendung als optische Aufheller**

(30) Priorität: **16.11.77 CH 14005/77**

(43) Veröffentlichungstag der Anmeldung: **30.05.79 Patentblatt 79/11**

(45) Bekanntmachung des Hinweises auf die Patenterteilung: **29.10.80 Patentblatt 80/22**

(84) Benannte Vertragsstaaten: **BE CH DE FR GB NL**

(56) Entgegenhaltungen:
**FR - A - 2 099 281**
**FR - A - 2 370 083**

(73) Patentinhaber: **HOECHST AKTIENGESELLSCHAFT**
**Zentrale Patentabteilung Postfach 80 03 20**
**D - 6230 Frankfurt/Main 80 (DE)**

(72) Erfinder: **Erckel, Rüdiger, Dr.**
**Staufenstrasse 16**
**D - 6239 Eppstein**
**Taunus (DE)**
**Schmidt, Erwin, Dr.**
**Am Flachsland 26**
**D - 6233 Kelkheim (Taunus) (DE)**
**Eckes, Helmut, Dr.**
**Kegelbahn 46**
**D - 6230 Frankfurt/Main 80 (DE)**
**Rösch, Günter**
**Hohlweg 17**
**D - 6232 Bad Soden am Taunus (DE)**

**EP 0 002 042 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Diamino-1,3,5-triazinylstilbenverbindungen, Verfahren zu deren Herstellung und ihre Verwendung als optische Aufheller

Aus der FR - AS - 2 370 083 sind bereits Triazinylstyryl-Verbindungen als optische Aufheller bekannt. Diese Verbindungen ergeben jedoch geringere Weißgrade im Vergleich zu den im folgenden beschriebenen erfindungsgemäßen Verbindungen. Weiterhin sind aus der FR - AS 2 099 281 Triazinylstilbenverbindungen als optische Aufheller bekannt, die im Triazinylring durch zwei Phenylgruppen substituiert sind. Diese Verbindungen haben aber nur eine eingeschränkte Verwendbarkeit, da sie sich nicht zur Aufhellung von Polyacrylnitril eignen, wie es bei den Verbindungen der vorliegenden Erfindung der Fall ist.

Die vorliegende Erfindung betrifft neue Diamino-1,3,5-triazinylstilbenzverbindungen, Verfahren zu deren Herstellung sowie deren Verwendung als optische Aufhellmittel für organische Materialien. Die neuen Diamino-1,3,5-triazinylstilbenverbindungen entsprechend der Formel

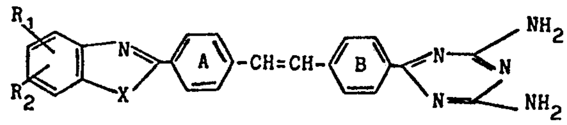

(1)

worin $R_1$ und $R_2$ gleich oder verschieden sein können und Wasserstoff oder nichtchromophore Reste der Reihe Fluor-, oder Chloratome, Phenyl-, Alkyl-, niedere Alkoxy-, niedere Dialkylamino-, niedere Trialkylammonium-, Acylaminogruppen oder gegebenenfalls funktionell abgewandelte Carboxy- oder Sulfogruppen bedeuten, wobei zwei benachbarte Reste $R_1$ und $R_2$ zusammen auch für eine niedere Alkylengruppe, einen ankondensierten Benzoring oder eine 1,3-Dioxypropylengruppe stehen können und die Ringe A und B noch weitere nicht-chromophore Substituenten aufweisen können, und X für O oder S steht.

Unter nicht-chromophoren Substituenten der Ringe A und B werden z.B. verstanden Alkyl mit 1 bis 12 Kohlenstoffatomen, Cyclohexyl, Phenyalkyl mit 1 bis 3 Kohlenstoffatomen im Alkylteil, unsubstituiertes oder mit 1 oder 2 Substituenten aus der Reihe Chlor, Methyl oder Methoxy substituiertes Phenyl, Alkoxy mit 1 bis 4 Kohlenstoffatomen, unsubstituiertes oder mit 1 oder 2 Substituenten aus der Reihe Chlor, Methyl oder Methoxy substituiertes Phenoxy, Chlor, Fluor, Brom, Cyano, —COOY, worin Y für Wasserstoff, ein salzbildendes Kation, Alkyl mit 1 bis 5 Kohlenstoffatomen oder Benzyl steht, CONY' ($Y_1'$), worin Y' für Wasserstoff, Alkyl mit 1 bis 6 Kohlenstoffatomen, Hydroxyalkyl mit 1 bis 4 Kohlenstoffatomen, Alkoxyalkyl mit 2 bis 8 Kohlenstoffatomen, Phenyl oder Benzyl und $Y_1'$ für Wasserstoff, Alkyl mit 1 bis 6 Kohlenstoffatomen, Hydroxyalkyl mit 1 bis 4 Kohlenstoffatomen oder Alkoxyalkyl mit 2 bis 8 Kohlenstoffatomen oder Y' und $Y_1'$ zusammen mit dem Stickstoff für einen Morpholin- oder Piperidinorest stehen, oder abgewandelte Sulfogruppen wie $—SO_2OY$, worin Y die vorstehend angegebene Bedeutung hat, $—SO_2NY'$ ($Y_1'$), worin Y' und $Y_1'$ die vorstehend angegebene Bedeutung haben, Alkylsulfonyl mit 1 bis 6 Kohlenstoffatomen, Benzylsulfonyl oder unsubstituiertes oder mit Chlor oder Methyl substituiertes Phenylsulfonyl, oder im Falle von zwei orthoständigen Substituenten auch Alkylen mit 3 oder 4 Kohlenstoffatomen oder 1.3-Butadienylen.

Bevorzugt sind solche Verbindungen der Formel

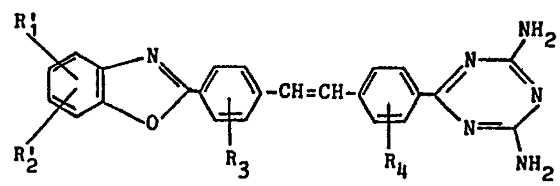

worin $R_1'$ und $R_2'$ unabhängig voneinander für Wasserstoff, Fluor-, oder Chloratome, Phenyl, Alkyl mit 1 bis 9 Kohlenstoffatomen, Alkoxy mit 1 bis 4 Kohlenstoffatomen, Carboxy, Carbalkoxy mit 1 bis 4 Kohlenstoffatomen im Alkoxyteil, Cyano oder abgewandelte Sulfogruppen stehen, wobei zwei benachbarte Reste $R_1$ und $R_2$ zusammen auch für eine niedere Alkylengruppe oder einen ankondensierten Benzoring stehen können und $R_3$ und $R_4$ unabhängig voneinander Wasserstoff, Halogen, Cyano, Alkoxy mit 1 bis 4 Kohlenstoffatomen oder abgewandelten Sulfogruppen wie oben bedeuten.

Besonders bevorzugt sind solche Verbindungen der Formel

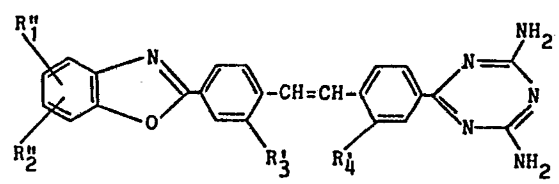

(3)

worin $R_1''$ und $R_2''$ unabhängig voneinander Wasserstoff, Fluor- oder Chloratome, Phenyl, Alkyl mit 1 bis 9 Kohlenstoffatome bedeuten und $R_3'$ und $R_4'$ unabhängig voneinander für Wasserstoff, Chlor oder Cyano stehen, sowie solche Verbindungen der Formel

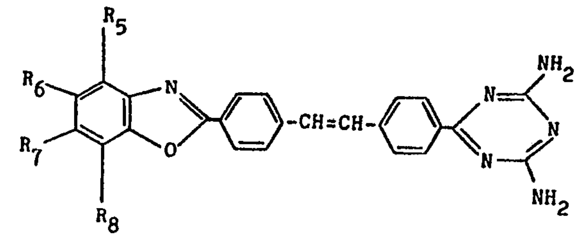

worin $R_5$, $R_6$, $R_7$ und $R_8$ Wasserstoff, $R_5$, $R_7$, $R_8$ Wasserstoff und $R_6$ Chlor, $C_1$—$C_4$-Alkyl oder Phenyl, $R_5$ und $R_7$ Wasserstoff und $R_6$ und $R_8$ $C_1$—$C_4$-Alkyl oder $R_5$ und $R_8$ Wasserstoff und $R_6$ und $R_7$ $C_1$—$C_4$-Alkyl bedeuten.

Von ganz besonderem Interesse sind Verbindungen der Formeln

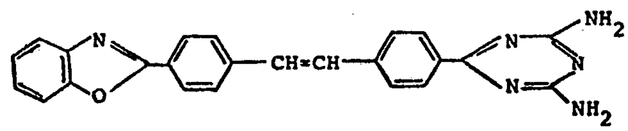 (4)

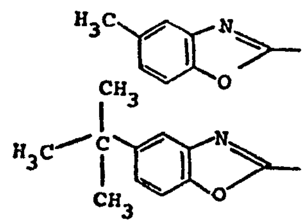 - " - (5)

- " - (6)

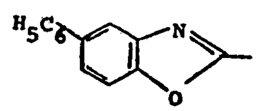 - " - (7)

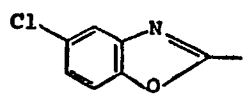 - " - (8)

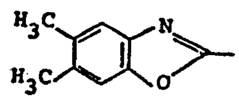 - " - (9)

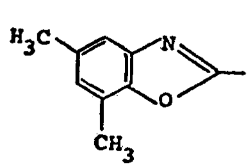 - " - (10)

Unter dem Begriff "functionell abgewandte Carboxy-gruppen" sind folgende zu verstehen:

Cyano-, Carbonsäureester-, Carbonsäureamid-, Mono- und Dialkylcarbonamid-gruppen. Der Begriff "niedere" umfaßt Gruppen mit 1—4 C-Atomen.

Die Diamino-1,3,5-triazinyl-stilbenverbindungen der Formel 1 werden in an sich bekannter Weise gemäß Org. Synth. Coll. Vol. IV, 78 aus Derivaten der Carbonsäuren

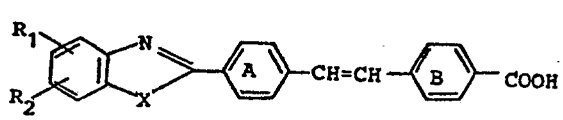 (13)

hergestellt, worin $R^1$, $R^2$, X, A und B die oben angegebene Bedeutung haben, beispielsweise aus dem Nitril durch Umsetzung mit Dicyandiamid in An- oder Abwesenheit basischer Katalysatoren oder aus dem entsprechenden Amidinsalz der Säure der Formel 13 durch Umsetzung mit Dicyandiamid oder

Biguanidsalzen oder durch Umsetzung des entsprechenden Chlorids der Säure der Formel 13 mit Biguanidsalzen oder durch Umsetzung des entsprechenden Nitrils der Säure der Formel 13 mit Harnstoff und Ammoniak.

Bevorzugte Darstellungsweise ist die Umsetzung des Nitrils der Carbonsäure (13) mit Dicyandiamid unter Zusatz basischer Katalysatoren in Gegenwart polarer Lösungsmittel bei Temperaturen von 70—250°C, gegebenenfalls unter Druck. Geeignete Lösungsmittel sind beispielsweise Methanol, Äthanol, i-Propanol, tert. Butanol, Cyclohexanol, Methylglykol, Methyldiglykol, Butylglykol, Diglykol, Dimethylformamid, Phosphorsäuretrisdimethylamid, Dimethylsulfoxid, Methylpyrrolidon.

Geeignete basische Katalysatoren sind Alkalicarbonate, -hydroxide, -amide und -alkoholate sowie primäre, sekundäre und tertiäre Amine oder quartäre Ammoniumbasen. Die Alkoholate können auch durch Auflösen von Alkalimetall, -amid oder -hydrid in dem also Lösungsmittel verwendeten Alkohol hergestellt werden.

Die als Ausgangsmaterial dienenden Carbonsäuren (13) bzw. die entsprechenden Nitrile und Säurechloride sind literaturbekannt bzw. können nach literaturbekannten Verfahren hergestellt werden (JA Sho 42—21013, US 3.577.411, DE—OS 20 00 027). Beispielsweise läßt sich die entsprechend substituierte Benzoxazoylstilbencarbonsäure nach an sich literaturbekannten Verfahren über das Säurechlorid in das Amid überführen und dieses ebenfalls nach an sich literaturbekannten Verfahren durch Umsetzung mit einem wasserabspaltenden Mittel zu dem entsprechenden Nitril umsetzen.

Eine weitere Möglichkeit zur Herstellung der Verbindungen der Formel 2 besteht darin, daß man eine Verbindung der Formel

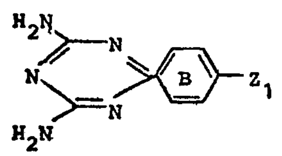

(14)

mit einer Verbindung der Formel

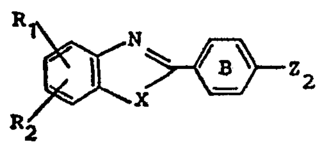

(15)

worin $R_1$, $R_2$, X, A und B die oben angegebene Bedeutung haben und eines der Symbole $Z_1$ und $Z_2$ eine —CHO-Gruppe und das andere eine Gruppierung der Formel

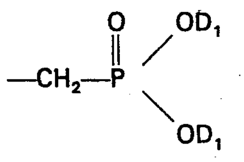 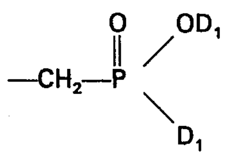 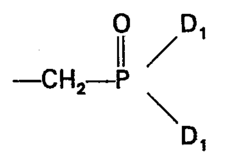 oder 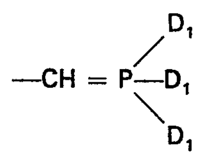

bedeuten, worin $D_1$ einen unsubstituierten oder substituierten Alkylrest, einen Arylrest, einen Cycloalkylrest oder einen Aralkylrest darstellt, umsetzt.

Man führt dieses Herstellungsverfahren vorteilhaft in indifferenten Lösungsmitteln durch. Als Beispiele hierfür seien Kohlenwasserstoffe wie Toluol und Xylol oder Alkohole wie Methanol, Äthanol, Isopropanol, Butanol, Glykole, Glykoläther wie 2-Methoxyäthanol, Hexanole, Cyclohexanol und Cyclooctanol, ferner Äther wie Diisopropyläther, Tetrahydrofuran und Dioxan sowie Dimethylsulfoxy, Formamid und N-Methylpyrrolidon genannt. Besonders geeignet sind polare organische Lösungsmittel wie Dimethylformamid und Dimethylsulfoxyd. Auch in wäßriger Lösung lassen sich einige der Umsetzungen durchführen.

Die Temperatur, bei der die Umsetzung durchgeführt wird, kann in weiten Grenzen schwanken. Sie wird bestimmt

$\alpha$) durch die Beständigkeit des verwendeten Lösungsmittels gegenüber den Reaktionssteilnehmern, insbesondere gegenüber den stark basischen Alkaliverbindungen,

$\beta$) durch die Reaktivität der Kondensationspartner und

$\gamma$) durch die Wirksamkeit der Kombination Lösungsmittel-Base als Kondensationsmittel

In der Praxis kommen hiernach im allgemeinen Temperaturen zwischen 10 und 100°C in Betracht, insbesondere, wenn Dimethylformamid oder Dimethylsulfoxid als Lösungsmittel verwendet werden. Der Temperatur-Vorzugsbereich liegt bei 20 bis 60°C.

Als stark basische Alkaliverbindungen kommen vor allem die Hydroxyde, Amide und Alkoholate (vorzugsweise solche primärer, 1 bis 4 Kohlenstoffatome enthaltender Alkohole) der Alkalimetalle in

Betracht, wobei aus ökonomischen Gründen solche des Lithiums, Natriums und Kaliums von vorwiegendem Interesse sind. Es können jedoch grundsätzlich und in besonderen Fällen auch Alkalisulfide und -carbonate, Arylalkaliverbindungen wie z.B. Phenyl-lithium oder stark basische Amine (einschließlich Ammoniumbasen) z.B. Trialkylammoniumhdyroxyde, mit Erfolg verwendet werden.

Die erfindungsgemäßen Verbindungen der Formel

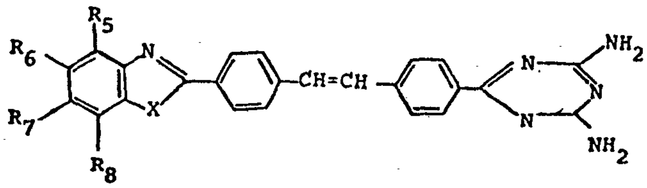

(16)

worin X, O oder S, $R_5$, $R_6$, $R_7$, $R_8$ Wasserstoff, Halogen, $R_5$ und $R_6$ oder $R_6$ und $R_7$ zusammen einen ankondensierten Benzolring und $R_6$ und $R_8$ Alkyl mit 1 bis 8 Kohlenstoffatomen bedeuten, können dadurch hergestellt werden, daß man eine Schiff'sche Base der Formel

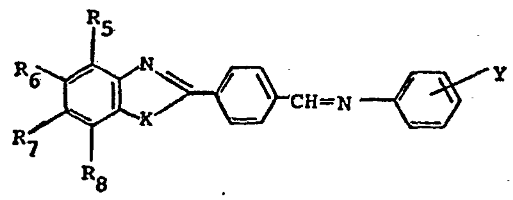

(17)

worin $R_5$, $R_6$, $R_7$, $R_8$ und X die vorstehende Bedeutung haben und Y Wasserstoff oder Chlor bedeutet, in einem polaren, neutralen bis basischen Lösungsmittel in Gegenwart einer stark basischen Alkaliverbindung, mit einer Methylverbindung der Formel

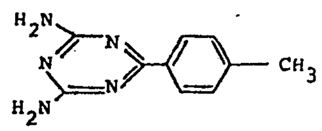

(18)

umsetzt.

Verbindungen der Formel (16) können auch dadurch hergestellt werden, daß man eine Methylverbindung der Formel

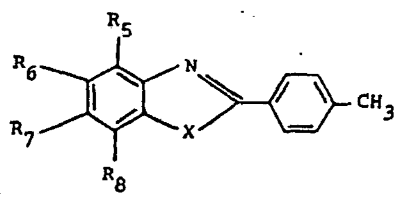

(19)

mit einer Schiff'schen Base der Formel

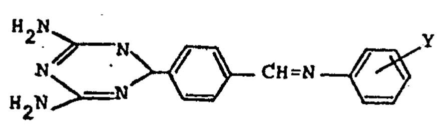

(20)

worin $R_5$, $R_6$, $R_7$, $R_8$,.S und Y die vorstehende Bedeutung haben, umsetzt.

Die methylengruppenhaltigen Verbindungen können mit den Anilen in Gegenwart eines geeignet starken polaren, neutralen bis alkalischen organischen Lösungsmittels umgesetzt werden, welches frei von Atomen, insbesondere Wasserstoffatomen, ist, die durch Alkalimetalle ersetzbar sind. Als Lösungsmittel kommen insbesondere Diäthylformamid, Hexamethylphosphorsäure-triamid, Tetramethylharnstoff infrage und insbesondere Dimethylformamid.

Für die Umsetzung ist weiterhin eine stark basische Alkalverbindung erforderlich. Je nach der Art des verwendeten Lösungsmittels und der Reaktionsfähigkeit des eingesetzten Anils sind dazu geeignet bestimmte Natriumalkoholate wie Natrium-t-butylat und besonders Kaliumverbindungen der Zusammensetzung

$$KOC_{m-1}H_{2m-1}$$

worin m eine ganze Zahl von 1 bis 6, vorzugsweise 2 bis 6, darstellt, wie z.B. Kaliumtertiär-butylat. Man kann außerdem die Reaktion auch mit KOH durchführen. Die Reaktionstemperatur liegt im allgemeinen

im Bereich zwischen 10 und 150°C. Bei besonders reaktionsfähigen Anilen gelingt die Reaktion auch schon bei Raumtemperatur.

Verbindungen der Formel (13) können auch hergestellt werden, indem man nach an sich bekannten Methoden hergestellte Stilbenverbindungen, die in 4-Stellen durch Cyano und in 4'Stellen durch eine gegebenenfalls abgewandelte Carboxylgruppe wie z.B. Säurechlorid, zu den entsprechenden Benzoxyzolyverbindungen in an sich bekannter Weise umsetzt. Die vorstehend definierten neuen Verbindungen zeigen in gelöstem oder feinverteiltem Zustand eine mehr oder weniger ausgeprägte Fluoreszenz. Sie können zum optischen Aufhellen der verschiedensten synthetischen, halbsynthetischen oder natürlichen organischen Materialen oder Substanzen, welche solche organischen Materialien enthalten, verwendet werden. Man verwendet sie insbesondere zum Aufhellen von Textilmaterial aus synthetischen Fasern wie Polyester, Polypropylen-, Polyamid- und besonders Polyacrylnitrilfasern. Die Applikation dieser optischen Aufheller erfolgt nach dem Ausziehverfahren oder dem Klotz-Thermofixierverfahren. Man kann die Aufheller aber auch bei der Herstellung der Fasern während der Polymerisation zusetzen oder aber den Spinnschmelzen oder Spinnlösungen zugeben, wobei die Spinnaufhellung von Polyacrylnitril ein bevorzugtes Anwendungsgebiet ist. Eine weitere Möglichkeit für die Applikation dieser Aufheller besteht darin, daß man sie einem Waschmittel zumischt, wobei das Textilmaterial während des Wachvorgangs aufgehellt wird.

Die Menge der erfindungsgemäß zu verwendenden neuen optischen Aufheller, bezogen auf das optisch aufzuhellende Material, kann in weiten Grenzen schwanken. Schon mit sehr geringen Mengen, in gewissen. Fällen z.B. solchen von 0,001 Gewichtsprozent, kann ein deutlicher und haltbarer Effekt erzielt werden. Es können aber auch Mengen bis zu etwa 0,8 Gewichtsprozent und gegebenenfalls bis zu eta 2 Gewichtsprozent zur Anwendung gelangen. Für die meisten praktischen Belange sind vorzugsweise Mengen zwischen 0,005 und 2, vorzugsweise 0,1 bis 0,5 Gewichtsprozent von Interesse.

Verbindungen der Formel (1), worin $R_3$ und/oder $R_4$ abgewandelte Sulfogruppen bedeuten, eignen sich besonders zum Aufhellen von Baumwolle und Polyamiden.

In den Beispielen sind Prozente immer Gewichtsprozente, Schmelz- und Siedepunkte sind, sofern nicht anders vermerkt, unkorrigiert.

Beispiel 1a

171 g 4'-Benzoxazolyl-2-stilbencarbonsäure werden in 1500 ml Toluol mit 428 g Thionylchlorid und 1 g Dimethylformamid 5 Stunden am Rückfluß gehalten. Das uberschüssige Thionylchlorid wird mit Toluol abdestilliert, der Kolbeninhalt auf 30°C abgekühlt und Ammoniak bis zur Sättigung eingeleitet. Unter weiterem Einleiten von Ammoniak wird noch 2 Stunden am Rückfluß gekocht. Nach dem Abkühlen wird abgesaugt, gewaschen und getrocknet.

Man erhält 146 g (86% d. Th.) 4'-Benzoxazolyl-2-stilbencarbonsäureamid der Formel

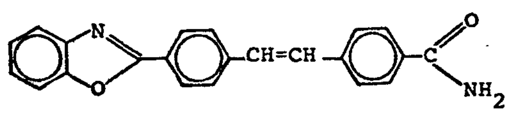

welches ohne weitere Reinigung in einer Mischung von 1400 g Thionylchlorid und 5 g DMF 6 Stunden am Rückfluß gerührt wird. Anschließend wird das Thionylchlorid bis zur Trockne abdestilliert, der Rückstand in Wasser verrührt, abgesaugt, neutral gewaschen und getrocknet. Man erhält 131 g (95% d. Th.) 4'-Benzoxazolyl-2-stilbencarbonsäurenitril der Formel

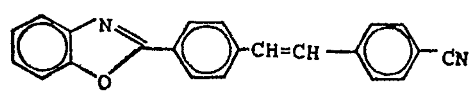

welches nach Umkristallisieren aus Methylglykol mit Tierkohle bei 240°C—242°C schmilzt.

IR: C=N 2222 $cm^{-1}$
UV: $\lambda_{max} = 358$ nm; $\varepsilon = 7{,}7 \times 10^4$ (in DMF)
Fluoreszenz (in DMF): $\lambda_{max} = 414$ nm

Beispiel 1b

32,2 g (0,1 Mol) 4'-Benzoxazolyl-2-stilben-4-carbonsäurenitril werden in 1000 ml Methylglykol mit 9,3 g Dicyandiamid und 2,0 g gepulvertem Kaliumhydroxyd 8 Stunden am Rückfluß gerührt. Anschließend wird bei Raumtemperatur abgesaugt, mit Methanol und anschließend mit Wasser neutral gewaschen und getrocknet. Man erhält 35 g (86,1% d. Th.) der Verbindung

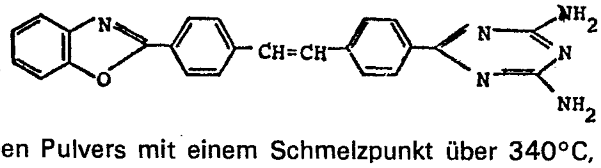

in Form eines hellgelben Pulvers mit einem Schmelzpunkt über 340°C,

Analyse: $C_{24}H_{18}N_6O$ [406,4]
ber. C 70,92 H 4,46 N 20,68
gef. C 71,1 H 4,6 N 20,4

UV (gemessen in Dimethylformamid)
$\lambda_{max} = 364$ nm; $\varepsilon = 7{,}13 \times 10^4$
Fluoreszenz (in DMF): 420 nm

Beispiel 2a

454, 6 g (3 Mol) p-Cyanbenzylchlorid und 560,0 ml (3,3 Mol) Triäthylphosphit werden 5 Stunden auf 140 bis 145°C erhitzt. Unter Abspaltung von Äthylchlorid entsteht nach einer Arbusow-Reaktion p-Cyan-benzyl-phosphonsäurediäthylester. Man kühlt das Reaktionsgemisch auf RT ab, legt Hockvakuum an und erhitzt langsam auf ca. 100°C Innentemperatur, um das überschüssige Triäthylphosphit abzudestillieren. Der rohe Phosphonsäureester kann ohne weitere Reinigung für die Folgereaktion eingesetzt werden. Ausbeute: 751,5 g (99%) Reinheit (GC): 96,1%ig

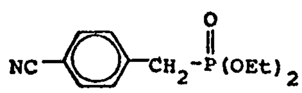

Beispiel 2b

112,2 g KOH-Pulver (2 Mol) werden unter Stickstoff in 1 Liter DMF suspendiert. Unter Kühlung mit Eiswasser tropft man eine Lösung von 128 g Phosphonester aus Beispiel 2a und 75 g p-Carboxybenzaldehyd (0,5 Mol) in 1 Liter DMF, so daß die Innentemperatur 25°C nicht übersteigt und rührt 12 Stunden bei Raumtemperatur nach. Das Reaktionsgemisch wird auf 5 Liter Eiswasser gegossen, das mit 200 ml konzentrierter Salzsäure angesäuert worden ist, erhitzt die Suspension kurz auf 90°C, saugt den Kristallbrei nach dem Erhalten ab, wäscht mit Wasser neutral und trocknet. Man erhält 105,5 g (85,9%) 4-Cyano-stilbencarbonsäure-(4') der Formel

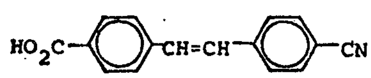

vom Schmp. 296—300°C, welche nach Umkristallisieren aus Benzoesäuremethylester unter Klärung mit Terikohle bei 301—302°C schmilzt.

Beispiel 2c

24,9 g (0,1 Mol) 4-Cyano-stilbencarbonsäure-(4') werden in 250 ml Chlorbenzol mit 9,2 ml (0,11 Mol) Thionylchlorid 2 Stunden auf 70—100°C erhitzt. Dann wird das überschüssige Thionylchlorid abdestilliert, 18,5 g (0,1 Mol) 4-Phenyl-2-aminophenol und 12,1 g (0,1 Mol) N,N-Dimethylanilin zugegeben und 45 Min. unter Rückfluß gekocht. Das Reaktionsgemisch wird abgekühlt, abgesaugt, mit Äthanol gewaschen und getrocknet. Man erhält 36,5 g (90%) rohe Acylverbindung vom Schmelzpunkt 262—264°C. Diese wird in 600 ml Trichlorbenzol nach Zugabe einer Spatelspitze p-Toluolsulfosäure 2 Stunden auf 212°C erhitzt, bei Raumtemperatur abgesaugt, mit Äthanol gewaschen und getrocknet. Man erhält 34,4 g (96,1%) 4-[5-Phenyl-benzoxazolyl-(2)]-4'-Cyanostilben der Formel

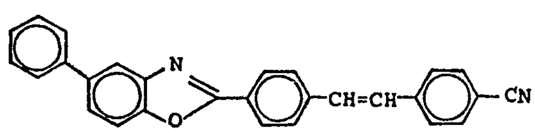

mit einem Schmelzpunkt (flüssig-kristalliner Übergang) von 237—239°C welche nach Umkristallisieren aus Dimethylformamid unter Klären mit Tierkohle einen flüssig-kristallinen Übergang bei 240—242°C zeigt, Klarpunkt 360°C.

Analyse: $C_{28}H_{18}N_2O$ [455,5]
ber. C 84,5 H 4,6 N 7,05
gef. C 84,2 H 4,6 N 6,9

UV (gemessen in Dimethylformamid)
$\lambda_{max} = 362$ nm; $\varepsilon = 7{,}17 \times 10^4$ü
Fluoreszenz (in DMF): 422 nm

beispiel 2

45,6 g (0,1 Mol) 4'-[5-Phenyl-benzoxazolyl-2]-stilben-4-carbonsäurenitril werden in 600 ml

Methyldiglykol mit 9,3 g Dicyandiamid und 2,0 g gepulvertem Kaliumhydroxid 8 Stunden am Rückfluß gerührt. Anschließend wird bei Raumtemperatur abgesaugt, mit Methanol und anschließend mit Wasser neutral gewaschen und getrocknet. Man erhält 39 g (81%) der Verbindung

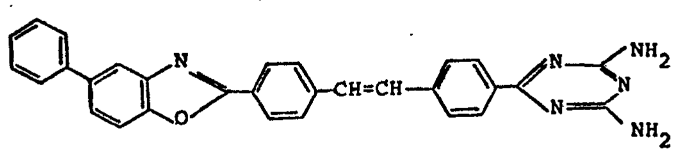

in Form eines hellgelben Pulvers mit einem Schmelzpunkt über 340°C

Analyse: $C_{30}H_{22}N_6O$ [482,6]
ber. C 74,67 H 4,60 N 17,52
gef. C 74,80 H 4,70 N 17,20

UV (in DMF): $\lambda_{max} = 368$ nm; $\varepsilon = 81700$
Fluoreszenz (in DMF): $\lambda_{max} = 427$ nm

Beispiel 3a

Arbeitet man wie in Beispiel 2c und setzt an Stelle des 4-Phenyl-2-aminophenol 12,3 g (0,1 Mol) 2-Amino-p-kresol ein, so erhält man 28,3 g (84% d. Th.) der Verbindung

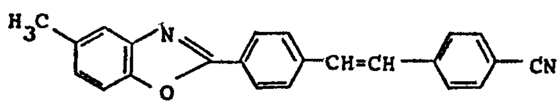

welche nach zweimaligem Umkristallisieren aus o-Dichlorbenzol unter Klärung mit Tierkohle einen flüssig-kristallinen Übergang bei 235°—237°C zeigt. Klarpunkt 300°C

Analyse: $C_{23}H_{16}N_2O$ [336,4]
ber. C 82,5 H 4,76 N 8,32
gef. C 81,80 H 4,90 N 8,1

UV (gemessen in Dimethylformamid)
$\lambda_{max} = 360$ nm; $\varepsilon = 6,95\ 10^4$
Fluoreszenz (in DMF): $\lambda_{max} = 420$ nm

Beispiel 3b

Setzt man das in Beispiel 3a erhaltene Nitril analog Beispiel 1b um, so erhält man 35,1 g (85% d. Th.) der Verbindung der Formel

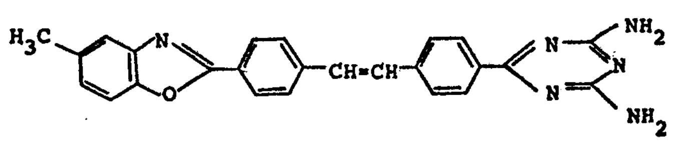

welche nach Umkristallisieren aus Dimethylformamid unter Klärung mit Tierkohle über 340° schmilzt.

analyse: $C_{25}H_{20}N_6O$ [420,5]
ber. C 71,40 H 4,79 N 19,98
gef. C 71,1 H 5,0 N 19,5

UV (in DMF): $\lambda_{max} = 366$ nm; $\varepsilon = 7,43 \times 10^4$
Fluoreszenz (in DMF): 428 nm

Beispiel 4a

Arbeitet man wie in Beispiel 2c und setzt an Stelle des 4-Phenyl-2-aminophenols 13,7 g 4,5-Dimethyl-2-aminophenol ein, so erhält man 28,2 g (81% d. Th.) der Verbindung

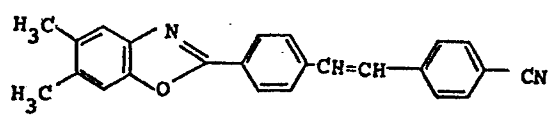

welche nach Umkristallisieren aus Benzoesäuremethylester unter Klärung mit Tierkohle einen flüssig-kristallinen Übergang bei 263—265°C zeigt und bei 302°C klar schmilzt.

UV (gemessen in Dimethylformamid)
$\lambda_{max} = 366$ nm; $\varepsilon = 6,46 \times 104$

Beispiel 4b

Setzt man das in Beispiel 3a erhaltene Nitril analog Beispiel 1b um, so erhält man 34 g (79% d. Th.) der Verbindung der Formel

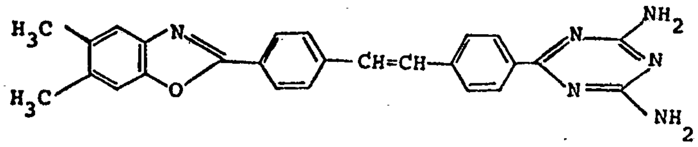

vom Schmelzpunkt 300°C.

UV (in DMF): $\lambda_{max} = 369$ nm
Fluoreszenz (in DMF): $\lambda_{max} = 428$ nm

Beispiel 5

4,02 g (0,02 Mol) 2,4-Dimaino-6-p-tolyl-1,3,5-triazin der Formel (Schmelzpunkt 245°C)

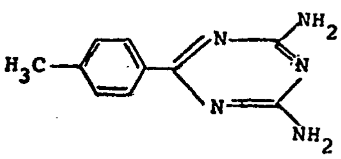

und 6,87 g (0,02 Mol) der Schiff'schen Base aus (2-(p-Formylphenyl)-benzoxazol der Formel

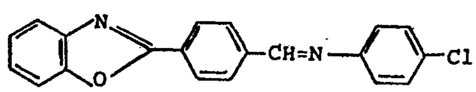

und 8,8 g Kalium-t-butylat werden in 200 ml Dimethylformamid 1 Stunde bei 25—35°C unter Stickstoff verrührt. Während der ersten 15 Minuten der Reaktion bestrahlt man das Reaktionsgemisch mit UV-Licht von Wellenlängen über 300 nm. Zur violetten Suspension gibt man dann 800 ml Methanol und saugt bei 0°C das ausgefallene Produkt ab, wäscht mit Methanol und Wasser. Das Rohprodukt wird in 100 ml Wasser verrührt mit Essigsäure auf pH 6—8 eingestellt, erneut abgesaugt, mit Wasser gewaschen und getrocknet.

Nach zweimaligem Umkristallisieren aus Dimethylformamid unter Klären mit Tierkohle erhält man 3,9 g (47,6% d. Th.) einer Verbindung wie in Beispiel 1b beschrieben.

Analog zu Beispiel 2c und 1b enthalt man folgende Verbindungen:

| | Verbindungen | Fp (°C) | Absorption (in DMF) max (nm) | | Fluoreszenz (in DMF) max (nm) |
|---|---|---|---|---|---|
| 6a | 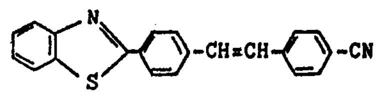 | 252–255 | – | – | – |
| 6b | 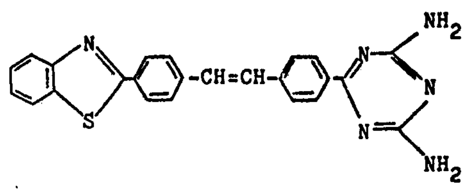 | >300 | 368 | $6,05 \times 10^4$ | 427 |
| 7a | 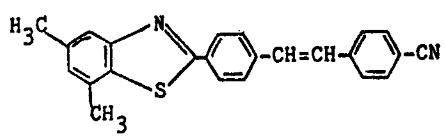 | 249–251 | 360 | $6,18 \times 10^4$ | 430 |
| 7b | 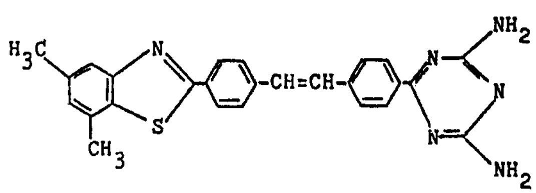 | 317–318 | 366 | $7,43 \times 10^4$ | 423 |

| | Verbindungen | Fp (°C) | Absorption (in DMF) max (nm) | | Fluoreszenz (in DMF) max (nm) |
|---|---|---|---|---|---|
| 8a | 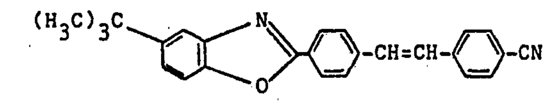 | 236–238 | 360 | 6,59 × $10^4$ | 420 |
| 8b | 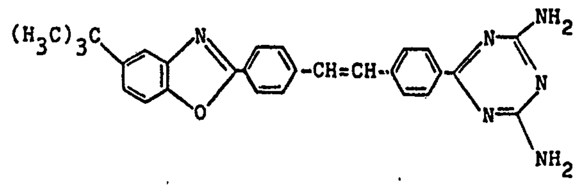 | >300 | 366 | 7,35 × $10^4$ | 422 |
| 9a | 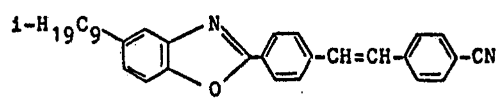 | 108–110 | 361 | 5,31 × $10^4$ | 422 |
| 9b | 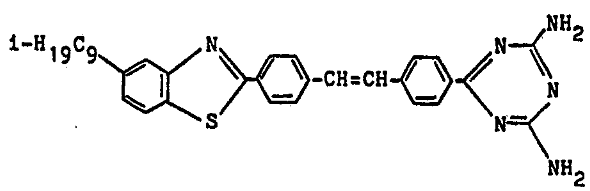 | 262 | 367 | 7,64 × $10^4$ | 423 |

| | Verbindungen | Fp (°C) | Absorption (in DMF) max (nm) | | Fluoreszenz (in DMF) max (nm) |
|---|---|---|---|---|---|
| 10a | 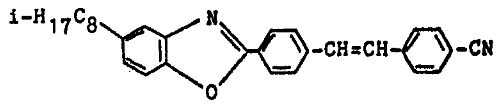 | 201–204 | 360 | 6,66 × 10⁴ | 424 |
| 10b | 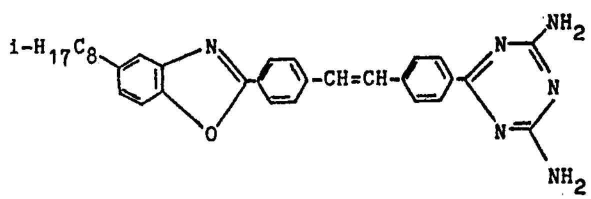 | | | | |
| 11a | 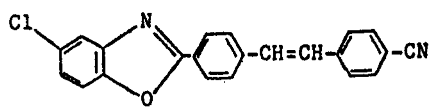 | 252–255 | 358 | 6,89 × 10⁴ | 412 |
| 11b | 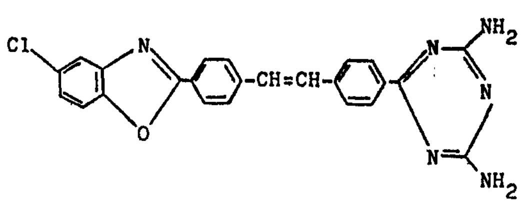 | | | | |

Für die Applikation auf Textilmaterial wurden je 100 mg der Verbindung aus Beispiel 1b (100%ig) in der Wärme in 10 ml Dimethylformamid gelöst und mit 5 ml eines Emulgators versetzt. Diese klare Lösung wurde unter Rühren in 85 ml einer wässrigen Lösung gegeben, die 100 ml/l des gleichen Emulgators enthält. Man erhielt so eine stabile Dispersion, die 1 g/l des optischen aufhellers enthält.

Anwendungsbeispiel 1

Ein Gewebe aus Polyesterfasern wurde mit der oben beschriebenen Dispersion getränkt und zwischen zwei Gummiwalzen auf eine Restfeuchte von ca. 70% abgequetscht. Dieses Gewebe wurde anschließend auf einem Spannrahmen einer Temperatur von 190°C während 30 Sekunden ausgesetzt. Nach der dabei verlaufenden Trocknung und Fixierung wies das Gewebe einen sehr hohen Weißgrad auf, der deutlich höher als der eines nicht behandelten Gewebes war.

Anwendungsbeispiel 2

Von der oben beschriebenen Dispersion wurde eine solche Menge abpipetiert, die 0,1% vom Warengewicht eines Polyamidgewebes entsprach. Diese Menge wurde in eine Flotte gegeben, die 1 g/l eines Bleichmittels enthielt. Nach einer Behandlung des Gewebes bei 98°C während 1 Stunde in einem Flottenverhältnis von 1:15 wurde gespült und getrocknet. Das so behandelte Gewebe wies einen sehr guten Weißgrad auf, der deutlich über einem unbehandelten Gewebe lag.

Anwendungsbeispiel 3

Es wurde eine Lösung des Aufhellers aus Beispiel 1b in Dimethylformamid hergestellt. Zu einer Spinnlösung aus Polyacrylnitril in Dimethylformamid wurde eine solche Menge der Aufhellerlösung zugegeben, daß, bezogen auf PAC, eine Menge von 200 ppm erreicht wurde. Die Spinnmasse wurde durch Spinndüsen gepreßt und das Lösungsmittel während des Abziehens der Fäden in der Trockenhitze verdampft. Nach dem Verstrecken der Faser wies diese einen brillanten Weißton auf, der deutlich höher war, als der von Fäden, die keinen Aufheller enthielten.

Anwendungsbeispiel 4

Auf Granulat aus Polyester wurde eine solche Menge an Aufhellersubstrat in einem Mischer aufgepudert, daß, bezogen auf PES, 200 ppm erreicht wurden. Auf einer Spritzmaschine wurden aus diesem Granulat Spritzteile hergestellt. Bei einer Spritztemperatur von 260°C wurden PES-Formteile erhalten, die einen ausgezeichneten Weißgrad von hoher Lichtechtheit aufwiesen.

**Patentansprüche**

1. Diamino-1,3,5-triazinyl-stilbenverbindungen der allgemeinen Formel

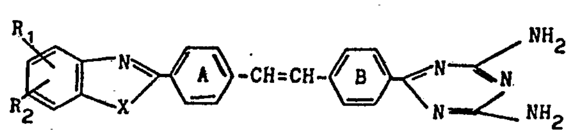

(1)

worin $R_1$ und $R_2$ gleich oder verschieden sein können und Wasserstoff oder nichtchromophore Reste der Reihe Fluor-, oder Chloratome, Phenyl-, Alkyl-, niedere Alkoxy-, niedere Dialkylamino-, niedere Trialkylammonium-, Acylaminogruppen oder gegebenenfalls funktionell abgewandelte Carboxy- oder Sulfogruppen bedeuten, wobei zwei benachbarte Reste $R_1$ und $R_2$ zusammen auch für eine niedere Alkylengruppe, einen ankondensierten Benzoring oder eine 1,3-Dioxypropylengruppe stehen können und die Ringe A und B noch weitere nicht-chromophore Substituenten aufweisen können, und X für O oder S steht.

2. Diamino-1,3,5-triazinyl-stilbenverbindungen nach Anspruch 1 der allgemeinen Formel

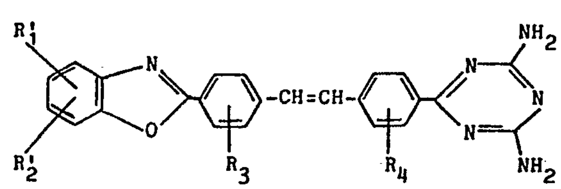

worin $R_1'$ und $R_2'$ unabhängig voneinander für Wasserstoff, Fluor-, oder Chloratome, Phenyl, Alkyl mit 1 bis 9 Kohlenstoffatomen, Alkoxy mit 1 bis 4 Kohlenstoffatomen, Carboxy, Caralkoxy mit 1 bis 4 Kohlenstoffatomen im Alkoxyteil, Cyano oder abgewandelte Sulfogruppen stehen, wobei zwei benachbarte Reste $R_1$ und $R_2$ zusammen auch für eine niedere Alkylengruppe oder einen ankondensierten Benzoring stehen können und $R_3$ und $R_4$ unabhängig voneinander Wasserstoff, Halogen, Cyano, Alkoxy mit 1 bis 4 Kohlenstoffatomen oder funktionell abgewandelten Sulfogruppen bedeuten.

3. Diamino-1,3,5-triazinyl-stilbenverbindungen nach Anspruch 1 der allgemeinen Formel

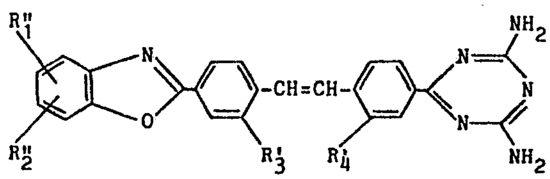

(3)

worin $R_1''$ und $R_2''$ unabhängig voneinander Wasserstoff, Fluor- oder Chloratome, Phenyl, Alkyl mit 1 bis 9 Kohlenstoffatome bedeuten und $R_3'$ und $R_4'$ unabhängig voneinander für Wasserstoff, Chlor oder Cyano stehen.

4. Diamino-1,3,5-triazinyl-stilbenverbindungen nach Anspruch 1 der allgemeinen Formel

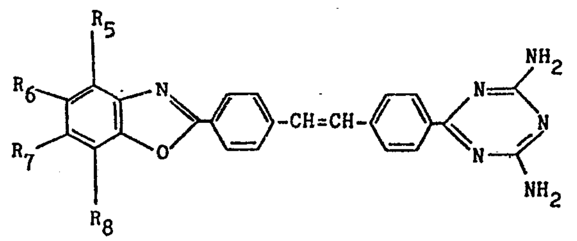

worin $R_5$, $R_6$, $R_7$ und $R_8$ Wasserstoff, $R_5$, $R_7$, $R_8$ Wasserstoff und $R_6$ Chlor, $C_1$—$C_4$-Alkyl oder Phenyl, $R_5$ und $R_7$ Wasserstoff und $R_6$ und $R_8$ $C_1$—$C_4$-Alkyl oder $R_5$ und $R_8$ Wasserstoff und $R_6$ und $R_7$ $C_1$—$C_4$-Alkyl bedeuten.

5. Verfahren zu Herstellung der Diamino-1,3,5-triazinyl-stilbenverbindungen nach Anspruch 1, dadurch gekennzeichnet, daß man ein Nitril der Formel

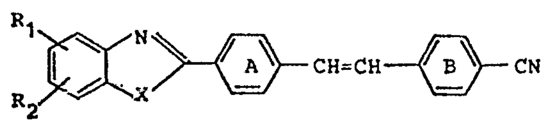

wobei $R_1$, $R_2$, X, A und B die in Anspruch 1 genannten Bedeutungen haben, mit Dicyanamid oder mit Harnstoff und Ammoniak umsetzt.

6. Verfahren zur Herstellung der Diamino-1,3,5-triazinyl-stilbenverbindungen nach Anspruch 1, dadurch gekennziechnet, daß man ein Amidinsalz der Formel

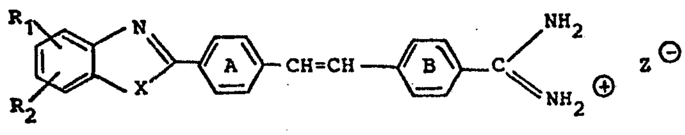

wobei $R_1$, $R_2$, X, A und B die in Ansprüchen 1 bis 3 genannten Bedeutungen haben und Z ein Anion bedeutet, mit Dicyanamid oder Biguanidsalzen umsetzt.

7. Verfahren zur Herstellung der Diamino-1,3,5-triazinyl-stilbenverbindungen nach Anspruch 1, dadurch gekennzeichnet, daß man ein Säurechlorid der Formel

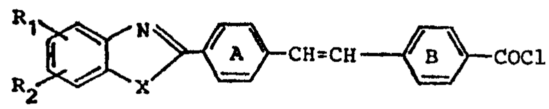

mit einem Biguanidsalz umsetzt.

8. Verfahren zur Herstellung der Diamino-1,3,5-triazinyl-stilbenverbindungen nach Anspruch 1, dadurch gekennzeichnet, daß man in einem inerten Lösungsmittel eine Verbindung der Formel

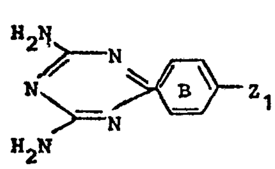 mit der Formel 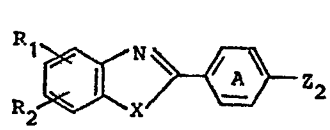

umsetzt, wobei $R_1$, $R_2$, X, A und B die in Ansprüchen 1 bis 3 angegebene Bedeutung haben und eines der Symbole $Z_1$ und $Z_2$ eine —CHO-Gruppe und das andere eine Gruppierung der Formel

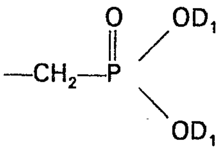 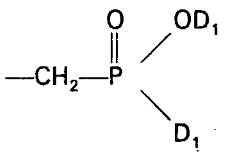 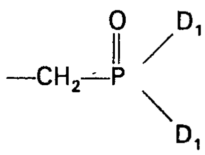 oder 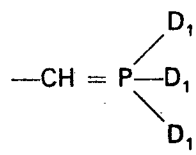

bedeuten, worin $D_1$ einen unsubstituierten oder substituierten Alkylrest, einen Arylrest, einen Cycloalkylrest oder einen Aralkylrest darstellt.

9. Verfahren zur Herstellung solcher Verbindungen nach Anspruch 1, die die allgemeine Formel

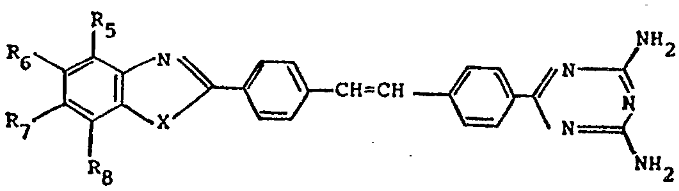

haben, worin X, O oder S, $R_5$, $R_6$, $R_7$, $R_8$ Wasserstoff, Halogen, $R_5$ und $R_6$ oder $R_6$ und $R_7$ zusammen einen ankondensierten Benzolring und $R_6$ und $R_8$ Alkyl mit 1 bis 8 Kohlenstoffatomen bedeuten, dadurch gekennzeichnet, daß man in eine polaren neutralen bis basischen Lösungsmittel und in Gegenwart einer stark basischen Alkaliverbindung eine Schiff'sche Base der Formel

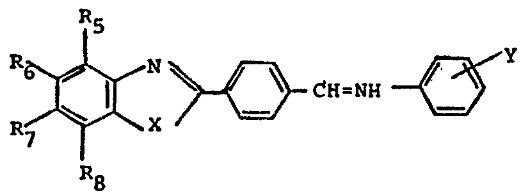

wobei $R_5$, $R_6$, $R_7$, $R_8$ und X die oben angegebene Bedeutung haben und Y Wasserstoff oder Chlor bedeutet, mit einer Methylverbindung der Formel

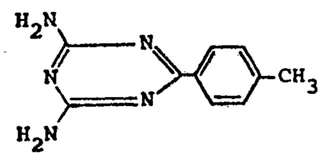

oder eine Schiff'sche Base der Formel

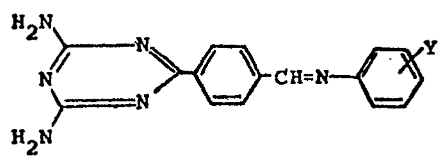

mit einer Methylverbindung der Formel

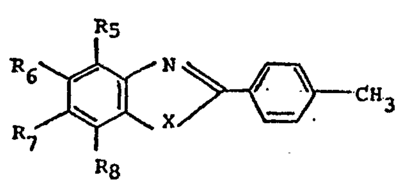

umsetzt, wobei $R_5$, $R_6$, $R_7$, $R_8$ und X die oben angegebene Bedeutung haben und Y Wasserstoff oder Chlor bedeutet.

10. Verwendung der Diamino-1,3,5-triazinyl-stilbenverbindungen nach Anspruch 1 als optische Aufheller.

**Claims**

1. Diamino-1,3,5-triazinylstilbene compounds of the formula

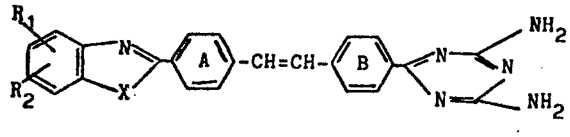

(1)

in which $R_1$ and $R_2$ may be identical or different and represent each hydrogen or non-chromophoric radicals selected from the group consisting of fluorine or chlorine atoms, phenyl, alkyl, lower alkoxy, lower dialkylamino, lower trialkylammonium, acyl amino or optionally functionally modified carboxy or sulfo groups, or two vicinal radicals $R_1$ and $R_2$ denote together lower alkylene, a fused benzo ring or 1,3-dioxypropylene, the rings A and B having optionally further non chromophoric substituents, and X is O or S.

2. Diamino-1,3,5-triazinylstilbene compounds as claimed in claim 1 of the formula

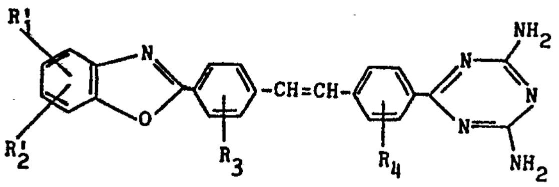

in which $R'_1$ and $R'_2$ independant from one another are hydrogen, fluorine, or chlorine atoms, phenyl, alkyl having from 1 to 9 carbon atoms, alkoxy having from 1 to 4 carbon atoms, carboxy, carbalkoxy having from 1 to 4 carbon atoms in the alkoxy moiety, cyano or modified sulfo groups or two vicinal radicals $R_1$ and $R_2$ denote together lower alkylene or a fused benzo ring and $R_3$ and $R_4$ independant from one another are hydrogen, halogen, cyano, alkoxy having from 1 to 4 carbon atoms or functionally modified sulfo groups.

3. Diamino-1,3,5-triazinylstilbene compounds as claimed in claim 1 of the formula

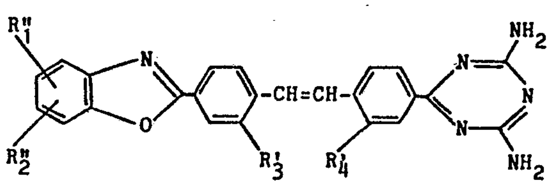

(3)

in which $R''_1$ and $R''_2$ independant from one another are hydrogen, fluorine or chlorine atoms, phenyl or alkyl having from 1 to 9 carbon atoms and $R'_3$ and $R'_4$ independant from one another are hydrogen, chlorine or cyano.

4. Diamino-1,3,5-triazinylstilbene compounds as claimed in claim 1 of the formula

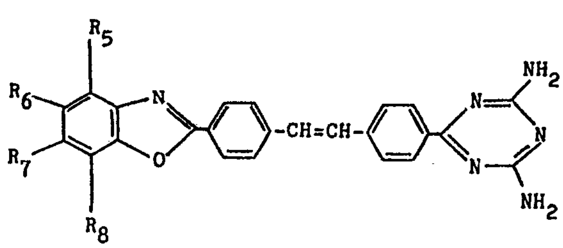

in which $R_5$, $R_6$, $R_7$ and $R_8$ each are hydrogen; $R_5$, $R_7$, $R_8$ each are hydrogen and $R_6$ is chlorine, $C_{1-4}$ alkyl or phenyl; $R_5$ and $R_7$ each are hydrogen and $R_6$ and $R_8$ each are $C_{1-4}$ alkyl or $R_5$ and $R_8$ each are hydrogen and $R_6$ and $R_7$ each are $C_{1-4}$ alkyl.

5. Process for the manufacture of the diamino-1,3,5-triazinylstilbene compounds as claimed in claim 1, which comprises reacting a nitrile of the formula

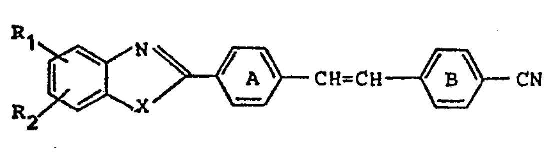

in which $R_1$, $R_2$, X, A and B are defined as in claim 1 with dicyanodiamide or with urea and ammonia.

6. Process for the manufacture of the diamino-1,3,5-triazinylstilbene compounds as claimed in claim 1, which comprises reacting an amidine salt of the formula

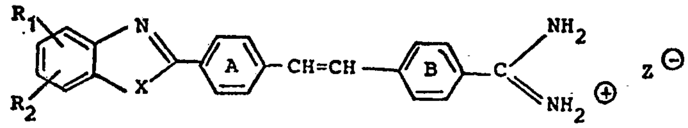

in which $R_1$, $R_2$, X, A and B are defined as in claims 1 to 3 and Z is an anion, with dicyanodiamide or biguanide salts.

7. Process for the manufacture of the diamino-1,3,5-triazinylstilbene compounds as claimed in claim 1, which comprises reacting an acid chloride of the formula

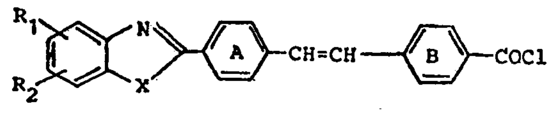

with a biguanide salt.

8. Process for the manufacture of the diamino-1,3,5-triazinylstilbene compounds as claimed in claim 1, which comprises reacting in an inert solvent a compound of the formula

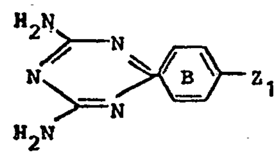

with a compound of the formula

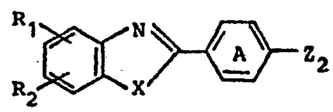

in which $R_1$, $R_2$, X, A and B are defined as in claims 1 to 3 and one of the symbols $Z_1$ and $Z_2$ is a —CHO— group and the other is a group of the formula

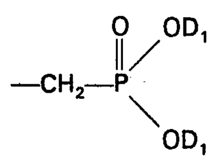 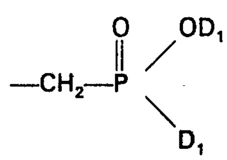 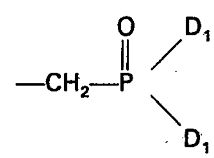 or 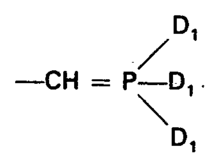

in which $D_1$ is unsubstituted or substituted alkyl, arylcycloalkyl or aralkyl.

9. Process for the manufacture of those compounds as claimed in claim 1, which have the formula

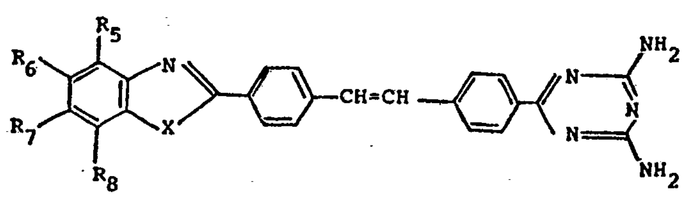

in which X is O or S, $R_5$, $R_6$, $R_7$, $R_8$ each are hydrogen or halogen, $R_5$ and $R_6$ and $R_7$ together denote a fused benzo ring and $R_6$ and $R_8$ each are alkyl having from 1 to 9 carbon atoms, which process comprises reacting in a polar, neutral to basic solvent and in the presence of a highly basic alkali metal compound a Schiff's base of the formula

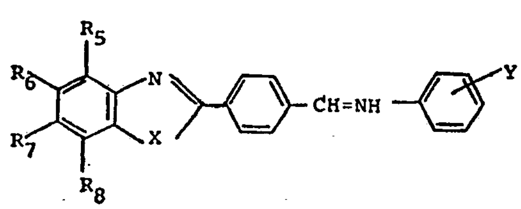

in which $R_5$, $R_6$, $R_7$, $R_8$ and X are defined as above and Y is hydrogen or chlorine, with a methyl compound of the formula

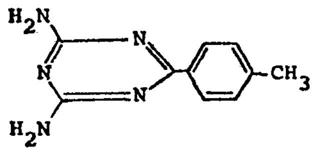

or reacting a Schiff's base of the formula

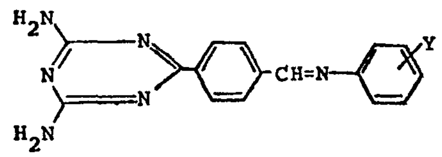

with a methyl compound of the formula

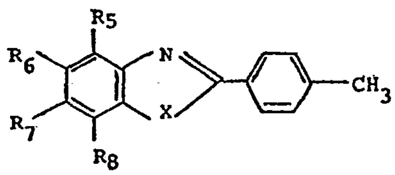

in which $R_5$, $R_6$, $R_7$, $R_8$ and X are defined as above and Y is hydrogen or chlorine.

10. Method for using the diamino-1,3,5-triazinylstilbene compounds as claimed in claim 1 as optical brighteners.

**Revendications**

1. (Diamino-triazine-1,3,5 yl)-stilbènes répondant à la formule générale:

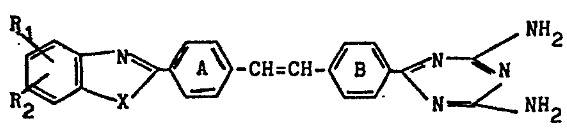

(1)

dans laquelle $R_1$ et $R_2$ représentent chacun, indépendamment l'un de l'autre, l'hydrogène ou un radical non chromophore appartenant à l'ensemble constitué par les atomes de fluor et de chlore, les radicaux phényles, alkyles, alcoxy inférieurs, dialkylamino inférieurs, trialkyl-ammoniums inférieurs et acylamino et les groupes carboxy et sulfo éventuellement sous la forme de dérivés fonctionnels, deux radicaux $R_1$ et $R_2$ voisins pouvant également représenter un radical alkylène inférieur, un noyau benzo condensé ou un radical propylène-dioxy-1,3, et les noyaux A et B pouvant porter d'autres substituants non chromophores, et X représente O ou S.

2. (Diamino-triazine-1,3,5 yl)-stilbènes selon la revendication 1, qui répondent à la formule générale:

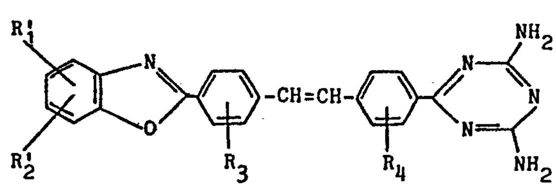

dans laquelle $R_1'$ et $R_2'$ représentent chacun, indépendamment l'un de l'autre, un atome d'hydrogène, de fluor ou de chlore, un radical phényl, un radical alkyle en $C_1$—$C_9$, un radical alcoxy en $C_1$—$C_4$, un groupe carboxy, un radical alcoxycarbonyle contenant de 1 à 4 atomes de carbone dans la partie alcoxy, un groupe cyano ou un groupe sulfo modifié, deux radicaux $R_1'$ et $R_2'$ voisins pouvant également former ensemble un radical alkylène inférieur ou un noyau benzo condensè, et $R_3$ et $R_4$ représentent chacun, indépendamment l'un de l'autre un atome d'hydrogène ou d'halogène, un groupe cyano, un radical alcoxy en $C_1$—$C_4$, ou un groupe sulfo modifié fonctionnellement.

3. (Diamino-triazine-1,3,5 yl)-stilbènes selon la revendication 1, qui répondent à la formule:

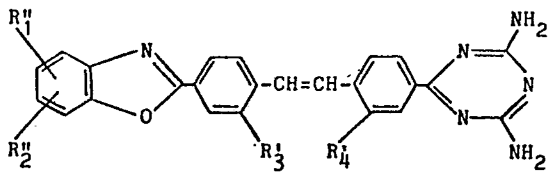

(3)

dans laquelle $R_1''$ et $R_2''$ représentent chacun, indépendamment l'un de l'autre, un atome d'hydrogène, de fluor ou de chlore, un radical phényle ou un radical alkyle contentant de 1 à 9 atomes de carbone, et $R_3'$ et $R_4'$ représentent chacun, indépendamment l'un de l'autre, un atome d'hydrogène ou de chlore ou un groupe cyano.

4. (Diamino-triazine-1,3,5 yl)-stilbènes selon la revendication 1, qui répondent à la formule:

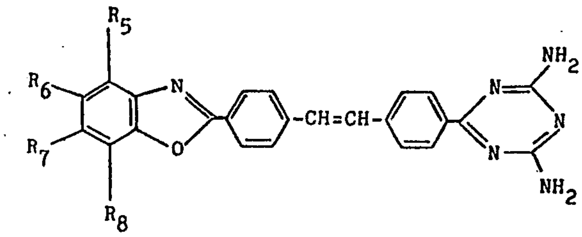

dans laquelle $R_5$, $R_6$, $R_7$ et $R_8$ représentent chacun un atome d'hydrogène, ou $R_5$, $R_7$ et $R_8$ représentent chacun l'hydrogène et $R_6$ représente le chlore, un radical alkyle en $C_1$—$C_4$ ou un radical phényle, ou $R_5$ et $R_7$ représentent chacun l'hydrogène et $R_6$ et $R_8$ chacun un radical alkyle en $C_1$—$C_4$, ou $R_5$ et $R_8$ représentent chacun l'hydrogène et $R_6$ et $R_7$ chacun un radical alkyle en $C_1$—$C_4$.

5. Procédé de préparation de (diamino-triazine-1,3,5 yl)-stilbènes selon la revendication 1, procédé caractérisé en ce qu'on fait réagir un nitrile de formule:

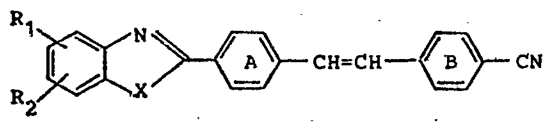

dans lequel $R_1$, $R_2$, X, A et B ont les significations données à la revendication 1, avec la cyano-guanidine ou avec l'urée et l'ammoniac.

6. Procédé de préparation de (diamino-triazine-1,3,5 yl)-stilbènes selon la revendication 1, caractérisé en ce qu'on fait réagir un sel d'amidine répondant à la formule:

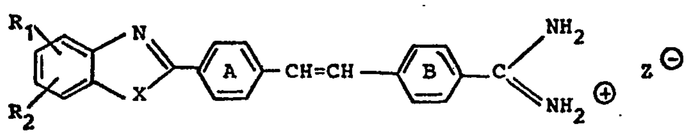

dans laquelle $R_1$, $R_2$, X, A et B ont les significations données dans les revendications 1 à 3 et Z représente un anion, avec la cyano-quanidine ou des sels du biguanide.

7. Procédé de préparation de (diamino-triazine-1,3,5 yl)-stilbènes selon la revendication 1, caractérisé en ce qu'on fait réagir un chlorure d'acide de formule:

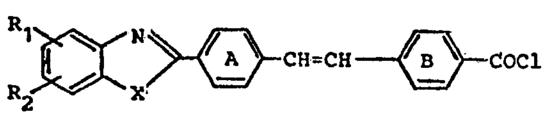

avec un sel du biguanide.

8. Procédé de préparation de (diamino-triazine-1,3,5 yl)-stilbènes selon la revendication 1, caractérisé en ce qu'on fait réagir, dans un solvant inerte, un composé de formule:

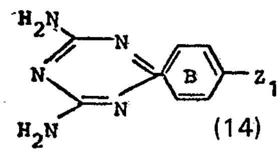

(14) avec un composé de formule:

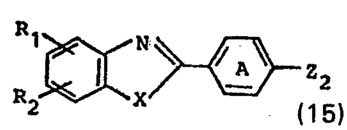

(15)

formules dans lesquelles $R_1$, $R_2$, X, A et B ont les significations données dans les revendications 1 à 3,

l'un des symboles $Z_1$ et $Z_2$ représente un groupe —CHO et l'autre représente un groupement répondant à l'une des formules:

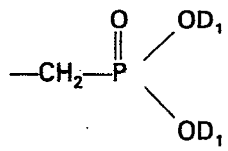 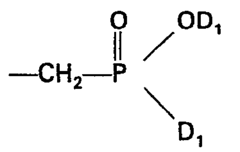 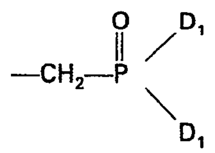 et 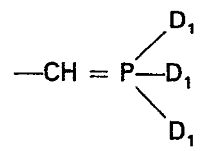

dans lesquelles $D_1$ représente un radical alkyle substitué ou non, un radical aryle, un radical cycloalkyle ou un radical aralkyle.

9. Procédé de préparation de composés selon la revendication 1 qui répondent à la formule générale:

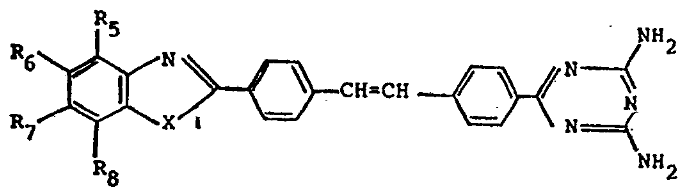

dans laquelle X représente O ou S, $R_5$, $R_6$, $R_7$, $R_8$ représentent chacun l'hydrogène ou un halogène, $R_5$ et $R_6$, ou $R_6$ et $R_7$, forment ensemble un noyau benzénique condensé, et $R_6$ et $R_8$ représentent chacun un radical alkyle contenant de 1 à 9 atomes de carbone, procédé caractérisé en ce qu'on fait réagir, dans un solvant polaire neutre à basique et en présence d'un composé de métal alcalin très basique, une base de Schiff répondant à la formule:

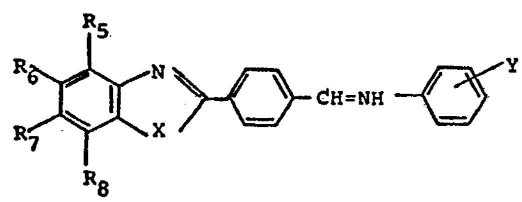

dans laquelle $R_5$, $R_6$, $R_7$, $R_8$ et X ont les significations données ci-dessus et Y représente l'hydrogène ou le chlore, avec un composé méthylique de formule:

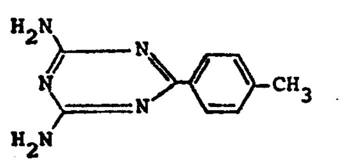

ou une base de Schiff de formule:

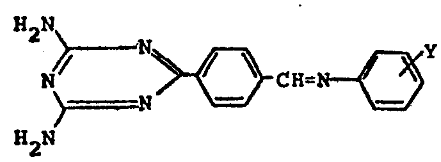

avec un composé méthylique de formule:

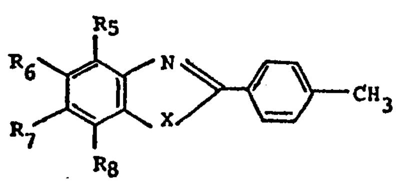

formules dans lesquelles $R_5$, $R_6$, $R_7$, $R_8$ et X ont les significations indiquées ci-dessus et Y représente l'hydrogène ou le chlore.

10. Utilisation des (diamino-triazine-1,3,5 yl)-stilbènes selon la revendication 1 comme azureurs optiques.